# FASCICULE DE BREVET EUROPEEN

(11) **EP 3 453 456 B1**
(45) Date de publication et mention de la délivrance du brevet: **08.04.2020**
(21) Numéro de dépôt: 18193939.8
(22) Date de dépôt: 12.09.2018
(51) Int. Cl.: B01L 3/02, A61M 5/315

(54) **PIPETTE DE MESURE**
MESSPIPETTE
MEASURING PIPETTE

(30) Priorité: 12.09.2017 FR 1758401
(43) Date de publication de la demande: 13.03.2019
(73) Titulaire: Erce Medical, 011000 Oyonnax (FR)
(72) Inventeur: DESPLANCHE, Christian, 01100 Arbent (FR)
(74) Mandataire: Chevalier, Renaud Philippe

(56) Documents cités:
- EP-A2- 0 326 361
- GB-A- 2 163 490
- US-A- 4 946 441
- US-A- 5 009 645

## Description

La présente invention concerne le domaine des pipettes de mesure pour le prélèvement d'une dose de produit et la délivrance dudit produit. Plus particulièrement, la présente invention concerne les pipettes de mesure graduées.

Classiquement une pipette de mesure graduée comprend un corps principal et un piston gradué agencé dans le corps principal. Le corps principal comprend une première extrémité ouverte par laquelle le piston est inséré et une deuxième extrémité présentant une ouverture plus petite que la première ouverture et configurée pour le prélèvement et la délivrance d'un produit. Le piston comprend une première extrémité dite extrémité de préhension et une deuxième extrémité opposée à la première extrémité et destinée à être en contact avec le produit.

Pour prélever un produit, le piston est entièrement inséré dans le corps principal jusqu'à être en butée contre la deuxième extrémité dudit corps principal, on immerge la deuxième extrémité du corps principal dans le produit et on tire le piston vers la première extrémité jusqu'à la graduation désirée. Le produit prélevé est généralement une substance liquide ou visqueuse ou pâteuse ou semi-liquide.

Pour délivrer le produit prélevé, on pousse le piston vers la deuxième extrémité du corps principal jusqu'à ce que ce dernier soit en butée contre ladite deuxième extrémité, le produit s'écoulant par l'ouverture de la deuxième extrémité du corps principal.

Pour figer la position du piston dans le corps principal à une certaine graduation, il existe des pipettes de mesure graduées qui utilisent un tampon ménagé sur le bout du piston (deuxième extrémité du piston), ledit tampon appliquant une force de frottement sur la surface interne de la paroi du corps principal, ce qui empêche le piston de bouger sans traction ou poussée de l'utilisateur. Cependant, ce type de pipette à tampon présente l'inconvénient de s'abimer dans le temps, le tampon s'use à force de frotter contre la surface interne de la paroi du corps principal et la force de frottement diminue jusqu'à être inexistante, ce qui conduit à des fuites et à un dosage imprécis.

Il existe également des pipettes présentant un corps principal réalisé dans matériau plastique élastiquement déformable et un piston dont la deuxième extrémité comprend un bourrelet. Sous la traction ou la poussée du piston dans le corps principal, la paroi du corps principal se déforme élastiquement localement au niveau du bourrelet du piston, ce qui retient temporairement le piston dans le corps principal au niveau de la graduation souhaitée par frottement et déformation. Cependant, ce système n'est pas sécurisé et à la moindre sollicitation du piston, ce dernier bouge engendrant un changement inopiné du dosage.

US 4 946 441 décrit une seringue incluant un piston dont le volume peut être réglé grâce à un anneau entourant le cylindre de la seringue et portant une vis. L'anneau, coulissant le long du cylindre, est positionné au niveau de la graduation désirée et la vis est serrée de manière à ce que sa pointe traverse la paroi du cylindre, formant ainsi un moyen de blocage du piston.

GB 2 163 490 décrit une seringue pour laquelle la quantité du liquide injecté est déterminée mécaniquement par la présence d'un moyen de bouchon fixé de manière amovible au plongeur, le moyen de bouchon et le plongeur étant amenés en contact l'un avec l'autre sous l'effet d'un engagement saillie-évidement.

US 5 009 645 décrit une seringue dont le piston inclut un rail sur lequel est placé de manière coulissante un moyen de blocage pouvant être bloqué à la position désirée.

EP 0 326 361 décrit une seringue dont le cylindre contient une goutte de résine durcie qui empêche d'en retirer le piston.

Les inconvénients majeurs des pipettes existantes sont de nécessiter une bonne visibilité de la graduation en toutes circonstances et de ne pas permettre le positionnement du piston à la graduation souhaitée avant le prélèvement du produit. Ainsi, dans des conditions telles que la nuit, où il y a peu de visibilité et où l'utilisateur présente une vigilance faible alors que le besoin de précision est primordial, des mauvais dosages ou des dosages imprécis peuvent se produire.

L'invention a pour but de remédier à tout ou partie des inconvénients précités et notamment à permettre un dosage précis et fiable en toutes circonstances.

À cet effet, l'invention a pour objet une pipette de mesure pour le prélèvement et l'administration d'une substance comprenant au moins :
- un corps principal comprenant une première extrémité ouverte et une deuxième extrémité opposée à la première extrémité, la deuxième extrémité comportant au moins un orifice d'entrée et de sortie de la substance, le corps principal présentant une paroi élastiquement déformable,
- un piston inséré dans le corps principal par la première extrémité ouverte dudit corps principal, le piston étant configuré pour être mobile au moins en translation dans le corps principal entre la première extrémité du corps principal et la deuxième extrémité du corps principal, le piston comprenant une première extrémité configurée pour créer une déformation élastique locale sur la paroi du corps principal,
caractérisée en ce que la pipette de mesure comprend en outre un organe de limitation positionné sur la surface externe de la paroi du corps principal et exerçant une force de compression déterminée pour empêcher la paroi du corps principal de se déformer et ainsi stopper la course du piston, la déformation créée par la première extrémité du piston venant en butée contre ledit organe de limitation.

L'avantage de la pipette de mesure selon l'invention est de pouvoir assurer une fiabilité de mesure à effectuer en toutes circonstances. En effet, il est possible avec l'organe de limitation de déterminer à l'avance un niveau auquel le piston doit s'arrêter, ce qui évite les surdosages ou les mauvais dosages, le niveau correspondant à une graduation particulière. En outre, l'organe de limitation met en exergue le niveau de dosage déterminé, ce qui permet une amélioration de la visibilité du dosage. Bien entendu, l'organe de limitation selon l'invention, permet de stopper la course du piston lorsque ledit piston est tiré en appliquant une force dite raisonnable ou normale sans forcer la résistance de l'organe de limitation.

Dans la présente invention, on entend par « déformation locale » une portion déformée localement de la paroi du corps principal de la pipette de mesure.

Selon une forme de réalisation de l'invention, le corps principal est creux.

Selon une forme de réalisation de l'invention, le piston comprend une deuxième extrémité opposée à la première extrémité et équipée d'un organe de préhension destiné à la manipulation du piston en traction ou en poussée. Selon une forme de réalisation de l'invention, l'organe de préhension se présente sous la forme d'une collerette s'étendant radialement depuis le corps du piston.

Selon une forme de réalisation de l'invention, l'organe de limitation est amovible et/ou mobile et/ou mobile.

Selon une forme de réalisation de l'invention, l'organe de limitation peut se présenter sous la forme d'une bague, d'une pince, ou tout autre élément comprenant au moins une portion en appui contre la surface externe de la paroi du corps principal. Préférentiellement, l'organe de limitation peut comprendre au moins deux portions d'appui positionnées sensiblement en regard l'une de l'autre configurées pour être en appui sur la surface externe de la paroi du corps principal et bloquer le piston.

Avantageusement, l'organe de limitation est adaptable sur n'importe quelle pipette existante.

Selon une forme de réalisation de l'invention, l'organe de limitation est amovible et/ou mobile.

Avantageusement, l'organe de limitation permet d'améliorer la lisibilité du réglage des doses : meilleure vision de la mire etmarquage différent au niveau du support. En effet, selon une forme de réalisation de l'invention l'organe de limitation étant agencé sur le corps principal, la préhension du piston n'obstrue pas la lisibilité due la graduation désirée.

Selon une première forme de réalisation de l'invention, l'organe de limitation est coulissant sur le corps principal.

Selon la première forme de réalisation de l'invention, l'organe de limitation comprend un trou conformé pour loger une portion du corps principal de la pipette de mesure.

Selon la première forme de réalisation de l'invention, l'organe de limitation comprend une première partie et une deuxième partie, la première partie coopérant avec la deuxième partie de préférence par clippage.

Selon la première forme de réalisation de l'invention, au moins une des parties, préférentiellement les deux parties, comprend un évidement.

Selon la première forme de réalisation de l'invention, au moins une des parties, préférentiellement les deux parties, comprend un ergot saillant.

Selon une forme de réalisation de l'invention, l'organe de limitation comprend au moins un organe de verrouillage formé par la coopération d'un évidement ménagé sur l'une des deux parties et d'un ergot saillant ménagé sur l'autre des deux parties.

Selon la première forme de réalisation de l'invention, la première partie de l'organe de limitation est déplacée en rotation par rapport à la deuxième partie 4b selon un axe de rotation X-X correspondant à l'axe longitudinal médian de l'organe de limitation ce qui permet de verrouiller l'organe de limitation sur le corps principal de la pipette de mesure.

Selon la première forme de réalisation de l'invention, en configuration déverrouillée, l'ergot saillant d'une des parties est en butée contre une première portion de l'évidement de l'autre des parties.

Selon la première forme de réalisation de l'invention, en configuration verrouillée, l'ergot saillant de l'une des parties est en butée contre une deuxième portion de l'évidement 4d2 créant une déformation de l'évidement.

Selon la première forme de réalisation de l'invention, la première portion de l'évidement a une section transversale plus grande que la section transversale de la deuxième portion. Préférentiellement, l'évidement a préférentiellement une forme de larme.

Selon une deuxième forme de réalisation de l'invention, l'organe de limitation comprend une portion principale comprenant un orifice conformé pour loger une portion du corps principal de la pipette de mesure.

Selon la deuxième forme de réalisation de l'invention, l'organe de limitation comprend au moins un organe de verrouillage, ce qui permet de verrouiller l'organe de limitation sur le corps principal.

Selon la deuxième forme de réalisation de l'invention, l'organe de limitation comprend en outre deux branches s'étendant depuis la portion principale et configurées pour coopérer l'une avec l'autre via l'organe de verrouillage.

Selon la deuxième forme de réalisation de l'invention, l'organe de verrouillage est ménagé entre les deux branches.

Selon la deuxième forme de réalisation de l'invention, l'organe de limitation comprend donc un organe de verrouillage, l'organe de verrouillage comprend une première partie équipant la première branche et une deuxième partie équipant la deuxième branche, la première partie et la deuxième partie étant conformées pour coopérer par complémentarité de forme. Préférentiellement, la première partie de l'organe de verrouillage est un crochet et la deuxième partie est un évidement conformé pour coopérer avec le crochet.

Selon la deuxième forme de réalisation de l'invention, l'organe de verrouillage se verrouille par pression des deux branches l'une vers l'autre puis relâchement.

Selon la deuxième forme de réalisation de l'invention, l'organe de verrouillage se déverrouille par pression des branches l'une vers l'autre puis traction des branches dans des directions opposées.

Selon une forme de réalisation de l'invention, la première extrémité du piston comprend un organe saillant. Selon une forme de réalisation de l'invention, l'organe saillant peut se présenter sous la forme d'un bourrelet circonférentiel ou tout autre élément créant une surépaisseur de la première extrémité du piston par rapport au corps du piston. Ainsi, en état monté, le piston est inséré dans le corps principal et le au moins un organe saillant déforme localement la paroi du corps principal assurant ainsi l'étanchéité.

Selon une forme de réalisation de l'invention, le corps principal de la pipette ou le piston est gradué. Selon une forme de réalisation de l'invention, la graduation peut être plane ou en relief.

L'invention sera mieux comprise, grâce à la description ci-après, qui se rapporte à des modes de réalisation selon la présente invention, donnés à titre d'exemples non limitatifs et expliqués avec référence aux figures schématiques annexées. Les figures schématiques annexées sont listées ci-dessous :
- la figure 1 est une vue en perspective de la pipette de mesure selon l'invention selon un premier mode de réalisation,
- la figure 2 est une vue en coupe longitudinale médiane selon l'axe X-X de la pipette de mesure illustrée en figure 1,
- la figure 3 est une en perspective de la pipette de mesure selon l'invention selon un deuxième mode de réalisation,
- la figure 4 est une vue en coupe longitudinale médiane selon l'axe X-X de la pipette de mesure illustrée en figure 3
- la figure 5 est une vue en perspective de la pipette de mesure selon l'invention en état de fonctionnement,
- la figure 6 est une vue en perspective d'un organe de limitation d'une pipette de mesure selon le premier mode de réalisation,
- la figure 7 est une vue en perspective éclaté de l'organe de limitation représenté en figure 6,
- la figure 8 est une vue de détail de l'organe de limitation illustré en figure 6, dans une configuration déverrouillée,
- la figure 9 est une vue de détail de l'organe de limitation illustré en figure 6, dans une configuration verrouillée,
- la figure 10 est une vue en perspective d'un organe de limitation d'une pipette de mesure selon le deuxième mode de réalisation,
- la figure 11 est une vue de détail de l'organe de limitation illustré en figure 10, dans une configuration déverrouillée,
- la figure 12 est une vue de détail de l'organe de limitation illustré en figure 10, dans une configuration verrouillée.

La pipette de mesure selon l'invention est destinée au prélèvement et l'administration par voie orale d'une substance préférentiellement médicamenteuse et ce, quel que soit le mode de réalisation.

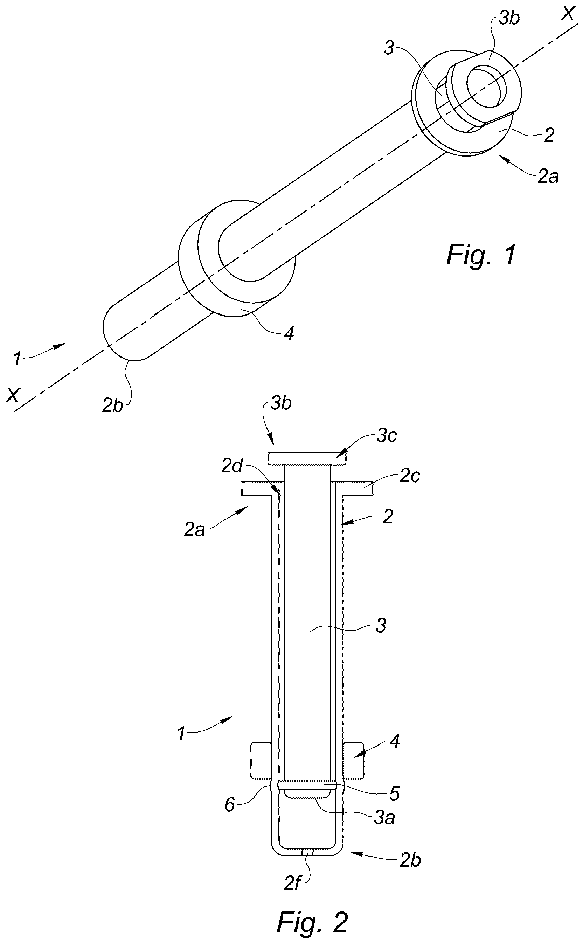

Quel que soit le mode de réalisation et comme illustré par exemple en figures 1, 2 et 5, la pipette de mesure 1 comprend un corps principal 2 allongé s'étendant selon un axe longitudinal X-X. Le corps principal 2 comprend une première extrémité 2a comprenant une ouverture 2d. Le corps principal 2 est creux. Le corps principal 2 comprend une deuxième extrémité 2b comportant au moins un orifice 2f d'entrée et de sortie de la substance. Le corps principal 2 présente une paroi élastiquement déformable comprenant une surface externe et une surface interne. La première extrémité 2a du corps principal comprend une collerette 2c s'étendant radialement autour de l'ouverture 2d du corps principal 2.

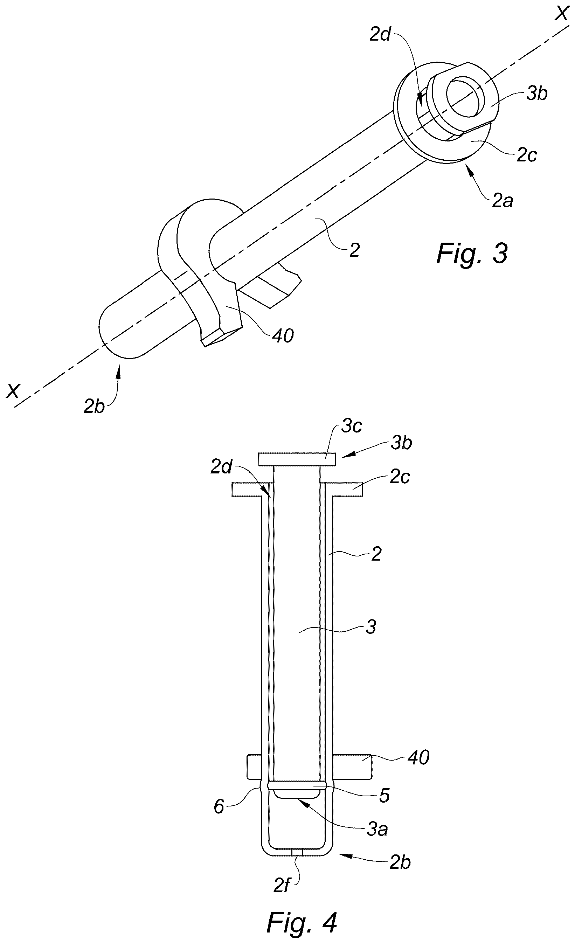

Quel que soit le mode de réalisation et comme illustré par exemple en figures 1 et 2, la pipette de mesure 1 comprend un piston 3conformé pour être inséré dans le corps principal 2 par la première extrémité 2a du corps principal 2. Aux figures 1 à 4, le piston 3 est illustré inséré dans le corps principal 2 de la pipette de mesure 1. Le piston 3 est configuré pour être mobile au moins en translation dans le corps principal 2 entre la première extrémité 2a et la deuxième extrémité 2b du corps principal 2. Le piston 3 comprend un corps, une première extrémité 3a destinée à venir au contact ou à proximité de la deuxième extrémité 2b du corps principal 2 lorsque le piston 3 est inséré dans le corps principal 2. La première extrémité 3a du piston 3 est conformée pour déformer élastiquement localement la paroi du corps principal en appliquant une force d'appui sur la surface interne de ladite paroi. Plus précisément, la première extrémité 3a du piston 3 comprend un organe saillant 5 qui peut prendre la forme d'un bourrelet circonférentiel comme illustré en figures 2 et 4. Bien entendu, l'invention n'est pas limitée à cet exemple et l'organe saillant 5 peut se présenter sous plusieurs formes telles qu'un ergot ou une pluralité d'ergots ou une surépaisseur discontinue ou continue ou autre. En état monté, la paroi du corps principal 2 présente donc une déformation 6 comme illustrée en figures 2 et 4.

Le piston 3 comprend une deuxième extrémité 3b opposée à la première extrémité 3a et équipée d'un organe de préhension 3c pour la manipulation du piston 3. Comme illustré aux figures 1 à 4, l'organe de préhension 3c se présente sous la forme d'une collerette s'étendant radialement depuis le corps du piston 3.

Quel que soit le mode de réalisation et comme illustré par exemple en figures 1 et 2, la pipette de mesure 1 comprend un organe de limitation 4, 40configuré pour stopper la course du piston 3 dans le corps principal 2à un niveau déterminé sur le corps principal 2, la déformation 6 locale du corps principal 2 venant en butée contre au moins une portion dudit organe de limitation 4, 40 comme illustré en figures 2 et 4. L'organe de limitation 4, 40 est positionné sur la surface externe de la paroi du corps principal 2.

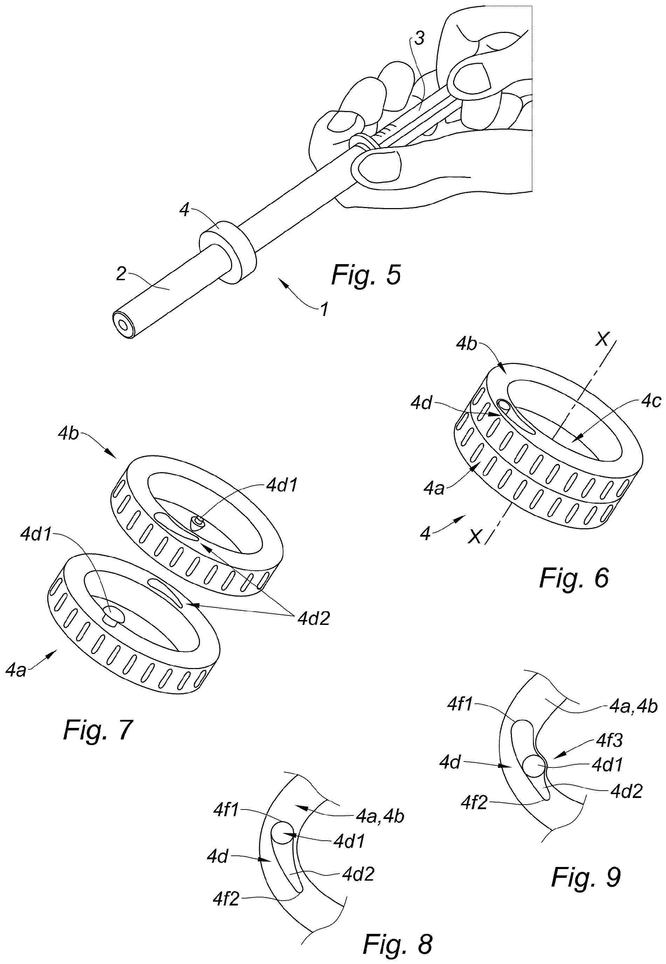

Conformément au premier mode de réalisation représenté aux figures 1 2, 6 à 9, l'organe de limitation 4 est une bague ou un anneau amovible et/ou mobile, coulissant sur le corps principal 2. L'organe de limitation 4 comprend un trou 4c conformé pour loger une portion du corps principal 2 de la pipette de mesure 1, l'organe de limitation 4 présente de par sa forme, au moins deux portions d'appui sur le corps principal 2. Comme illustré en figures 6 à 9, l'organe de limitation 4 comprend une première partie 4a, une deuxième partie 4b. La première partie 4a est clippée à la deuxième partie 4b comme illustré en figure 6. Plus particulièrement, chaque partie 4a, 4b comprend un évidement 4d2 et un ergot saillant 4d1, formant par coopération un organe de verrouillage 4d. Dans l'exemple illustré en figure 6, l'organe de limitation 4 comprend deux organes de verrouillage 4d mais il pourrait aussi ne comprendre qu'un seul organe de verrouillage 4d ou bien une pluralité d'organes de verrouillage 4d.

Dans l'exemple illustré aux figures 6 et 7, l'évidement 4f d'une partie coopère par clippage avec l'ergot saillant 4d de l'autre partie. En outre, pour verrouiller le clippage, on fait tourner la première partie 4a par rapport à la deuxième partie 4b selon l'axe de rotation X-X, comme illustré aux figures 8 et 9. Dans une configuration déverrouillée représentée en figure 8, l'ergot saillant 4d1 est en butée contre une première portion 4f1 de l'évidement 4d2 et dans une configuration verrouillée représentée en figure 9, l'ergot saillant 4d1 se déplace et vient en butée contre une deuxième portion 4f2 de l'évidement 4d2 créant une déformation 4f3 qui, par frottement, bloque l'ergot saillant 4d1 dans la deuxième portion 4f2. L'évidement 4d2 a préférentiellement une forme de larme selon laquelle la première portion 4f1 présente une section transversale plus grande que la section transversale de la deuxième portion 4f2.

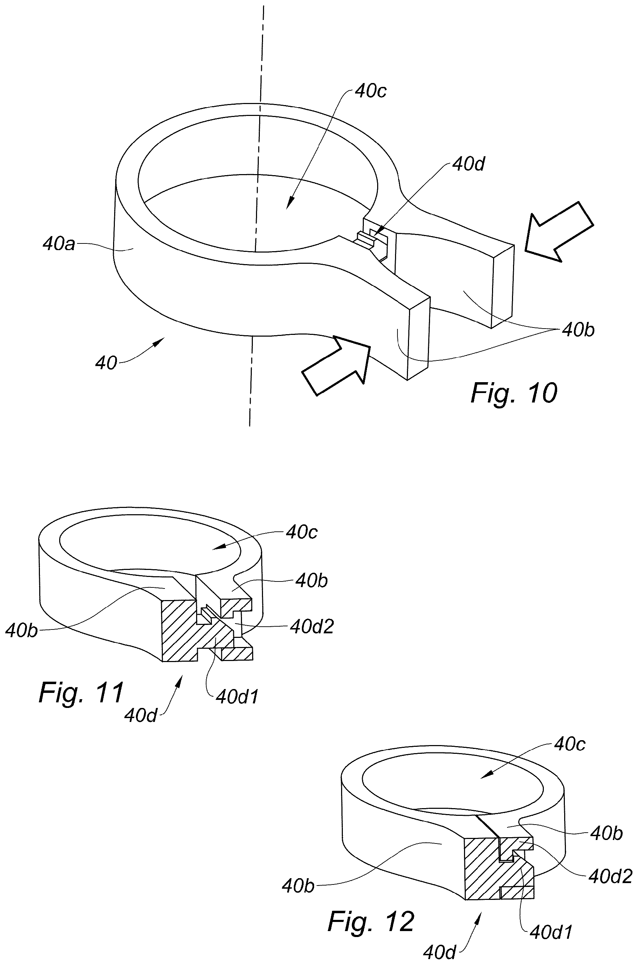

Selon le deuxième mode de réalisation représenté aux figures 3, 4 et 10 à 12, l'organe de limitation 40 est une pince ou un cerclage amovible et/ou mobile. L'organe de limitation 40 présente de par sa forme, au moins deux portions d'appui sur le corps principal 2. En figure 10, l'organe de limitation 40 comprend une portion principale 40a comprenant un orifice 40c conformé pour loger une portion du corps principal 2 de la pipette de mesure 1. L'organe de limitation 40 comprend en outre deux branches 40b s'étendant de puis la portion principale 40a et configurée pour coopérer l'une avec l'autre grâce à un organe de verrouillage 40d. L'organe de limitation 40 comprend donc un organe de verrouillage 40d ménagé entre les deux branches 40b, comme illustré en figure 10. L'organe de verrouillage 40d comprend une première partie 40d1 équipant la première branche 40b et une deuxième partie 40d2 équipant la deuxième branche 40b, comme représenté notamment en figure 11. Dans l'exemple illustré aux figures 10 à 12, la première partie 40d1 de l'organe de verrouillage 40d est un crochet et la deuxième partie 40d2 est un évidement conformé pour coopérer avec le crochet. Bien entendu, cet exemple et non limitatif et la première 40d1 et la deuxième partie 40d2 de l'organe de verrouillage 40d et même l'organe de verrouillage 40d peuvent prendre une autre forme sans que cela ne sorte du cadre de l'invention. L'organe de verrouillage 40d de l'organe de limitation selon le deuxième mode de réalisation, se verrouille par pression des deux branches 40b l'une vers l'autre puis relâchement (figure 12) et se déverrouille par pression des branches l'une vers l'autre puis traction des branches 40b dans des directions opposées.

En cours d'utilisation, la graduation est visible sans être gênée par la préhension du corps principal 2 et/ou du piston 3 comme illustré en figure 5. En effet, la main de l'utilisateur est sur le piston 3 et non sur la graduation.

Quel que soit le mode de réalisation de la pipette de mesure 1, on utilise la pipette de mesure 1 selon l'invention comme décrit ci-après. À vide, le piston 3 est en appui contre la deuxième extrémité 2b du corps principal 2. On place ensuite l'organe de limitation 4, 40 au niveau de la graduation souhaitée, et on verrouille l'organe de limitation 4, 40 au moyen de l'organe de verrouillage 4d, 40d soit par rotation (premier mode de réalisation) soit par clippage (deuxième mode de réalisation). Puis, on place la pipette en immersion dans la substance à prélever et l'on tire sur le piston 3 dont le bourrelet circonférentiel ou organe saillant 5 déforme la paroi du corps principal 2 jusqu'à être bloqué par l'organe de limitation 4, 40. La dose de substance est alors prélevée et il ne reste plus qu'à l'administrer en poussant le piston 3 vers la deuxième extrémité 2b du corps principal 2.

Il est à noter que le positionnement de l'organe de limitation 4, 40 sur le corps principal 2 de la pipette de mesure 1 peut être avantageusement fait à l'avance lorsque l'utilisateur a plus de visibilité pour le faire ou quand sa vigilance est plus accrue et que le prélèvement de substance et son administration peuvent être réalisées plus tard, en toute sécurité car l'organe de limitation 4, 40 est déjà mis en place et verrouillé.

Bien entendu, l'invention n'est pas limitée à ces deux modes de réalisation et l'organe de limitation 4 peut se présenter sous la forme de tout autre organe ou élément comprenant au moins une portion en appui, préférentiellement au moins deux portions, contre la surface externe de la paroi du corps principal 2.

Bien entendu, l'invention n'est pas limitée aux modes de réalisation décrits et représentés aux figures annexées. Des modifications restent possibles, notamment du point de vue de la constitution des divers éléments, sans sortir pour autant du domaine de protection de l'invention, qui est défini par les revendications.

## Revendications

1. Pipette de mesure (1) pour le prélèvement et l'administration d'une substance comprenant au moins :
- un corps principal (2) comprenant une première extrémité (2a) ouverte et une deuxième extrémité (2b) opposée à la première extrémité (2a), la deuxième extrémité (2b) comportant au moins un orifice (2f) d'entrée et de sortie de la substance, le corps principal (2) présentant une paroi élastiquement déformable,
- un piston (3) inséré dans le corps principal (2) par la première extrémité (2a) ouverte dudit corps principal (2), le piston (3) étant configuré pour être mobile au moins en translation dans le corps principal (2) entre la première extrémité (2a) du corps principal (2) et la deuxième extrémité (2b) du corps principal (2), le piston (3) comprenant une première extrémité (3a) créeant une déformation(6) élastique locale sur la paroi du corps principal (2),
**caractérisée en ce que** la pipette de mesure (1) comprend en outre un organe de limitation (4, 40) positionné sur la surface externe de la paroi du corps principal (2) et exerçant une force de compression déterminée pour empêcher la paroi du corps principal (2) de se déformer et ainsi stopper la course du piston (3), la déformation créée par la première extrémité (3a) du piston (3) venant en butée contre ledit organe de limitation (4,40).

2. Pipette de mesure selon la revendication 1, dans laquelle la première extrémité (3a) du piston (3) comprend un organe saillant (5).

3. Pipette de mesure selon la revendication 2, dans laquelle l'organe saillant (5) se présente sous la forme d'un bourrelet circonférentiel.

4. Pipette de mesure selon l'une quelconque des revendications précédentes, dans laquelle l'organe de limitation (4) est amovible et/ou mobile.

5. Pipette de mesure selon l'une quelconque des revendications précédentes, dans laquelle l'organe de limitation (4) est un élément comprenant au moins une portion en appui contre la surface externe de la paroi du corps principal (2).

6. Pipette de mesure selon l'une quelconque des revendications précédentes, dans laquelle l'organe de limitation (4) comprend au moins deux portions d'appui positionnées en regard l'une de l'autre et configurées pour être en appui sur la paroi du corps principal (2) et bloquer le piston (3).

7. Pipette de mesure selon l'une quelconque des revendications précédentes, dans laquelle le corps principal (2) de la pipette de mesure (1) ou le piston (3) est gradué.

8. Pipette de mesure selon l'une quelconque des revendications précédentes, dans laquelle l'organe de limitation (4) est une bague ou un anneau.

9. Pipette de mesure selon l'une quelconque des revendications 1 à 7, dans laquelle l'organe de limitation (40) est une pince ou un cerclage.

10. Pipette de mesure selon l'une quelconque des revendications 1 à 9, dans laquelle l'organe de limitation (4, 40) comprend au moins un organe de verrouillage (4d, 40d) configuré pour verrouiller la position de l'organe de limitation sur le corps (2) de la pipette (1).

## Patentansprüche

1. Messpipette (1) zum Entnehmen und Verabreichen einer Substanz, mindestens Folgendes umfassend:
- einen Hauptkörper (2), ein erstes offenes Ende (2a) und ein zum ersten Ende (2a) entgegengesetztes zweites Ende (2b) umfassend, wobei das zweite Ende (2b) mindestens eine Öffnung (2f) zum Eintreten und Austreten der Substanz beinhaltet, wobei der Hauptkörper (2) eine elastisch verformbare Wand aufweist,
- einen Kolben (3), der durch das erste offene Ende (2a) des Hauptkörpers (2) in den Hauptkörper (2) eingeführt wird, wobei der Kolben (3) konfiguriert ist, um in dem Hauptkörper (2) zwischen dem ersten Ende (2a) des Hauptkörpers (2) und dem zweiten Ende (2b) des Hauptkörpers (2) mindestens translationsbeweglich zu sein, wobei der Kolben (3) ein erstes Ende (3a) umfasst, das eine lokale elastische Verformung (6) an der Wand des Hauptkörpers (2) erstellt,
**dadurch gekennzeichnet, dass** die Messpipette (1) weiter ein Begrenzungsorgan (4, 40) umfasst, das an der Außenoberfläche der Wand des Hauptkörpers (2) positioniert ist und eine bestimmte Verdichtungskraft ausübt, um die Wand des Hauptkörpers (2) daran zu hindern, sich zu verformen und somit den Hub des Kolbens (3) anzuhalten, wobei die von dem ersten Ende (3a) des Kolbens (3) erstellte Verformung auf Anschlag an das Begrenzungsorgan (4, 40) kommt.

2. Messpipette nach Anspruch 1, wobei das erste Ende (3a) des Kolbens (3) ein hervorstehendes Organ (5) umfasst.

3. Messpipette nach Anspruch 2, wobei sich das hervorstehende Organ (5) in Form eines umlaufenden Wulstes darstellt.

4. Messpipette nach einem der vorstehenden Ansprüche, wobei das Begrenzungsorgan (4) abnehmbar und/ oder beweglich ist.

5. Messpipette nach einem der vorstehenden Ansprüche, wobei das Begrenzungsorgan (4) ein Element ist, das mindestens einen Abschnitt an der Außenoberfläche der Wand des Hauptkörpers (2) anliegend umfasst.

6. Messpipette nach einem der vorstehenden Ansprüche, wobei das Begrenzungsorgan (4) mindestens zwei Anlageabschnitte umfasst, die einander gegenüber positioniert sind, und konfiguriert sind, um an der Wand des Hauptkörpers (2) anzuliegen und den Kolben (3) zu blockieren.

7. Messpipette nach einem der vorstehenden Ansprüche, wobei der Hauptkörper (2) der Messpipette (1) oder der Kolben (3) graduiert ist.

8. Messpipette nach einem der vorstehenden Ansprüche, wobei das Begrenzungsorgan (4) ein Ring oder Reif ist.

9. Messpipette nach einem der Ansprüche 1 bis 7, wobei das Begrenzungsorgan (40) eine Zange oder eine Umreifung ist.

10. Messpipette nach einem der Ansprüche 1 bis 9, wobei das Begrenzungsorgan (4, 40) mindestens ein Verriegelungsorgan (4d, 40d) umfasst, das konfiguriert ist, um die Position des Begrenzungsorgans am Körper (2) der Pipette (1) zu verriegeln.

## Claims

1. A measuring pipette (1) for the sampling and the administration of a substance comprising at least:
- one main body (2) comprising a first open end (2a) and a second end (2b) opposite to the first end (2a), the second end (2b) including at least one orifice (2f) for the inlet and outlet of the substance, the main body (2) having an elastically deformable wall,
- one piston (3) inserted into the main body (2) by the first open end (2a) of said main body (2), the piston (3) being configured to be movable at least in translation in the main body (2) between the first end (2a) of the main body (2) and the second end (2b) of the main body (2), the piston (3) comprising a first end (3a) creating a local elastic deformation (6) on the wall of the main body (2),
**characterized in that** the measuring pipette (1) further comprises a limiting member (4, 40) positioned on the outer surface of the wall of the main body (2) and exerting a determined compressive force to prevent the wall of the main body (2) from deforming and thus to stop the piston stroke (3), the deformation created by the first end (3a) of the piston (3) abutting against said limiting member (4, 40).

2. The measuring pipette according to claim 1, wherein the first end (3a) of the piston (3) comprises a protruding member (5).

3. The measuring pipette according to claim 2, wherein the protruding member (5) is in the form of a circumferential bead.

4. The measuring pipette according to any one of the preceding claims, wherein the limiting member (4) is removable and/or movable.

5. The measuring pipette according to any one of the preceding claims, wherein the limiting member (4) is an element comprising at least one portion bearing against the outer surface of the wall of the main body (2).

6. The measuring pipette according to any one of the preceding claims, wherein the limiting member (4) comprises at least two bearing portions positioned opposite to each other and configured to bear on the wall of the main body (2) and block the piston (3).

7. The measuring pipette according to any one of the preceding claims, wherein the main body (2) of the measuring pipette (1) or the piston (3) is graduated.

8. The measuring pipette according to any one of the preceding claims, wherein the limiting member (4) is a ring or an annulus.

9. The measuring pipette according to any one of claims 1 to 7, wherein the limiting member (40) is a clamp or a strap.

10. The measuring pipette according to any one of claims 1 to 9, wherein the limiting member (4, 40) comprises at least one locking member (4d, 40d) configured to lock the position of the limiting member on the body (2) of the pipette (1).
